(19) Europäisches Patentamt
European Patent Office
Office européen des brevets

(11) Veröffentlichungsnummer: **0 094 566**
**B1**

(12) # EUROPÄISCHE PATENTSCHRIFT

(45) Veröffentlichungstag der Patentschrift: **12.03.86**

(51) Int. Cl.⁴: **C 07 D 405/12**, A 61 K 31/415, A 61 K 31/34, A 01 N 43/50

(21) Anmeldenummer: **83104415.1**

(22) Anmeldetag: **05.05.83**

(54) **Dibenzofuranoxyalkyl-imidazoliumsalze, Verfahren zu ihrer Herstellung und ihre Verwendung als Mikrozide.**

(30) Priorität: **13.05.82 DE 3217963**

(43) Veröffentlichungstag der Anmeldung: **23.11.83 Patentblatt 83/47**

(45) Bekanntmachung des Hinweises auf die Patenterteilung: **12.03.86 Patentblatt 86/11**

(84) Benannte Vertragsstaaten: **AT BE CH DE FR GB IT LI NL SE**

(56) Entgegenhaltungen: **US - A - 4 199 346**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Meyer, Norbert, Dr., Stahlbuehlring 155a, D-6802 Ladenburg (DE)**
Erfinder: **Sauter, Hubert, Dr., Neckarpromenade, D-6800 Mannheim 1 (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

EP 0 094 566 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Dibenzofuranoxyalkylimidazoliumsalze, Verfahren zu ihrer Herstellung und mikrozide, d. h. fungizide und bakterizide Mittel, die diese Verbindungen als Wirkstoffe enthalten.

Es ist bekannt, N-Trichlormethylthiotetrahydrophthalimid als Fungizid zu verwenden („Chemical Week", 21. Juni 1972, S. 46). Seine Wirksamkeit befriedigt nicht in allen Fällen.

Der Erfindung lag die Aufgabe zugrunde, eine neue Fungizidklasse zu finden, die hochwirksam ist und gleichzeitig ein breites Anwendungsspektrum als Bakterizid besitzt.

Es wurde nun gefunden, dass Dibenzofuranoxyalkylimidazoliumsalze der Formel I

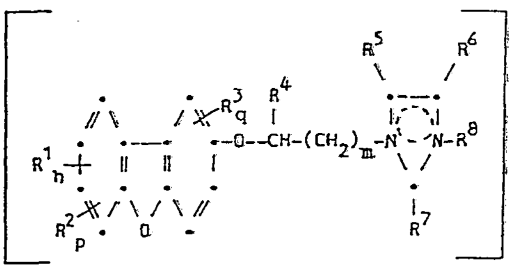 $X^{\ominus}$ (I)

in der

$R^1$, $R^2$ und $R^3$ gleich oder verschieden sind und Wasserstoff, Halogen, eine ggf. halogensubstituierte Alkyl- oder Alkoxygruppe mit 1 bis 4 Kohlenstoffatomen, Cyan oder Nitro,

$R^4$, $R^5$, $R^6$ und $R^7$ unabhängig voneinander Wasserstoff oder Alkyl mit 1 bis 4 Kohlenstoffatomen bedeuten, und

$R^8$ einen Alkyl-, Alkenyl- oder Alkinylrest mit bis zu 6 Kohlenstoffatomen oder eine der Gruppen

$$-CH_2-Ar,\ -CH_2-\underset{|}{\overset{R^4}{C}}=CH-Ar$$

oder $-(CH_2)_{2\text{-}5}-O-Ar$

bedeutet, wobei Ar für 1- und 2-Naphthyl, Biphenylyl oder Phenyl steht und der Phenylrest durch einen oder zwei gleiche oder verschiedene Reste aus der Gruppe Fluor, Chlor, Brom, Nitro, Trifluormethyl, Cyan oder Alkyl, Alkenyl und Alkoxy mit bis zu 5 Kohlenstoffatomen substituiert sein kann, und

m die Zahlen 1, 2, 3 und 4,

n, p und q die Zahlen 0, 1, 2 oder 3, und

x das Anion einer einbasigen, nichtphytotoxischen Säure oder ein Äquivalent einer mehrbasigen, nichtphytotoxischen Säure bedeuten, eine gute fungizide und bakterizide Wirksamkeit zeigen.

Als Substituenten für $R^1$, $R^2$ und $R^3$ in der Formel I kommen Halogen, also Fluor, Brom, Iod, ferner die Nitro- und die Cyangruppe sowie ggf. durch Halogen substituierte Alkyl- oder Alkoxyreste mit 1 bis 4 Kohlenstoffatomen, wie Methyl, Ethyl, Isopropyl, tert.-Butyl, Methoxy, Ethoxy, Trifluormethyl, Difluormethoxy, in Betracht.

$R^4$, $R^5$, $R^6$ und $R^7$ bedeuten Wasserstoff oder $C_{1\text{-}4}$-Alkyl, beispielsweise Methyl oder Ethyl; $R^8$ bedeutet $C_{1\text{-}6}$-Alkyl, -Alkenyl oder -Alkinyl, beispielsweise Methyl, Ethyl, n-Propyl, n-Butyl, Isobutyl, n-Pentyl, Isopentyl, n-Hexyl, Vinyl, Allyl, 2-Buten-1-yl, 3-Methyl-2-buten-1-yl, Propargyl, oder die Reste

$$-CH_2-Ar,\ -CH_2-\underset{R^4}{\underset{|}{C}}=CH-Ar$$

oder $-(CH_2)_{\overline{m+1}}O-Ar$.

Ar bedeutet beispielsweise 1- und 2-Naphthyl, Biphenylyl, Phenyl, 4-Fluorphenyl, 3- und 4-Chlorphenyl, 4-Bromphenyl, 3,4-Dichlorphenyl, 2,4-Dichlorphenyl, 2,6-Dichlorphenyl, 2-Methyl-4-chlorphenyl, 4-Methoxyphenyl, 4-Ethoxyphenyl, 4-Methylphenyl, 4-Isopropylphenyl, 4-tert.-Butylphenyl, 4-Nitrophenyl, 4-Trifluormethylphenyl, 2,4-Dimethylphenyl und 4-Cyanphenyl.

m bedeutet die Zahlen 1, 2, 3 und 4,

n, p und q beudenten die Zahlen 0, 1, 2 und 3.

X bedeutet das Anion einer einbasigen, nichtphytotoxischen Säure oder ein Äquivalent einer mehrbasigen, nichtphytotoxischen Säure, beispielsweise von Chlorwasserstoffsäure, Bromwasserstoffsäure, Iodwasserstoffsäure, Methoschwefelsäure, Phenylsulfonsäure, p-Methylphenylsulfonsäure, p-Dodecylphenylsulfonsäure, Salpetersäure oder ein Äquivalent des Schwefelsäureanions.

Man erhält die Dibenzofuranoxyalkylimidazoliumsalze der Formel I durch Umsetzung von

a) Verbindungen der Formel II

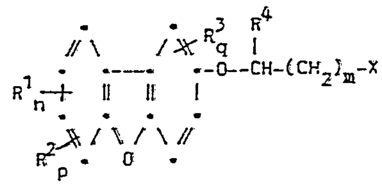 (II)

in der $R^1$, $R^2$, $R^3$, $R^4$, X, m, n, p und q die oben angegebenen Bedeutungen haben, mit einem Imidazolderivat der Formel III

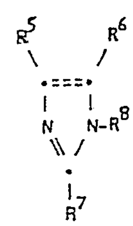 (III)

in der $R^5$, $R^6$, $R^7$ und $R^8$ die oben angegebenen Bedeutungen haben, oder

b) Verbindungen der Formel IV

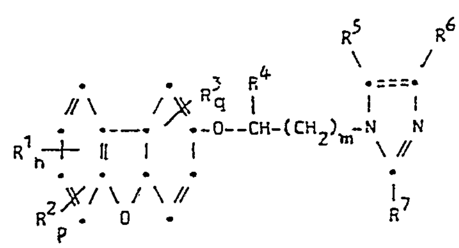 (IV)

in der $R^1$, $R^2$, $R^3$, $R^4$, $R^5$, $R^6$, $R^7$, m, n, p und q die oben angegebenen Bedeutungen haben, mit einer Verbindung der Formel

$$R^8-X$$

in der $R^8$ und X die oben angegebenen Bedeutungen haben.

Die Reaktionen a) und b) werden, ggf. in Gegenwart eines inerten Lösungs- oder Verdünnungsmittels, bei einer Temperatur im Bereich zwischen 20 und 150, vorzugsweise zwischen 30 und 140° C, durchgeführt. Der Ausgangsstoff der Formel II wird zweckmässigerweise mit einem bis 10fachen molaren Überschuss des Imidazolderivates der Formel III umgesetzt.

Als bevorzugte, gegenüber den Reaktionsteilnehmern inerte Lösungs- oder Verdünnungsmittel können beispielsweise aliphatische oder aromatische, ggf. halogenierte Kohlenwasserstoffe, wie Pentan, Cyclohexan, Benzol, Toluol, Xylol, Chlorbenzol, Dichlorbenzole, aliphatische Ketone, wie Aceton, Methylethylketon, Diethylketon oder Cyclopentanon, Ether, wie Diethylether, Dimethoxyethan, Tetrahydrofuran oder Dioxan, Ester, wie Essigsäureethylester, Nitrile, wie Acetonitril, Amide, wie Dimethylformamid, Dimethylacetamid und N-Methylpyrrolidon, oder Gemische dieser Lösungsmittel verwendet werden.

Die Ausgangsstoffe der Formel II können leicht nach bekannten Verfahren hergestellt werden, beispielsweise durch Veretherung von Dibenzofuran-3-olen mit aliphatischen Dihalogeniden, wie 1,2-Dibromethan, 1,3-Dichlorpropan, 1-Chlor-3-Brompropan, 1,3-Dibrompropan, 1-Chlor-4-brombutan oder 1,4-Dibrombutan, bevorzugt in siedendem Methylethylketon, Diethylketon oder Cyclopentanon in Gegenwart von mindestens äquivalenten Mengen Natriumcarbonat oder Kaliumcarbonat (vgl. Houben-Weyl, „Methoden der Organischen Chemie", Bd. 6/3, S. 54-59, Georg Thieme-Verlag, Stuttgart, 1965). Alternativ können Dibenzofuran-3-oxyalkanole mit Thionylchlorid oder mit Phosphortribromid zu Verbindungen der Formel II umgesetzt werden (vgl. „Rec. trav. Chim.", *76*, 129 bis 146 [1957]).

Die Imidazolderivate der Formel IV lassen sich nach bekannten Verfahren herstellen, beispielsweise nach dem Schema

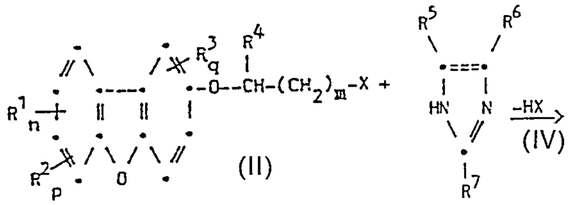

(II) (IV)

(V)

durch Alkylierung von kommerziell zugänglichen Imidazolen der Formel V in der $R^5$, $R^6$ und $R^7$ die oben genannten Bedeutungen haben, mit den Verbindungen der Formel II. Die Reaktionsbedingungen entsprechen hierbei denen der Umsetzung a).

Die neuen Imidazoliumsalze der Formel I besitzen, sofern $R^4$ nicht Wasserstoff bedeutet, ein chirales, mit dem Ligand $R^4$ gebundenes Kohlenstoffatomen und ggf. noch weitere Chiralitätszentren in $R^8$. Nach üblichen Methoden können die optisch reinen Enantiomeren bzw. die Diastereomeren erhalten werden. Die Erfindung erfasst auch diese Verbindungen in reiner Form oder als Mischungen. Als Fungizide und Bakterizide sind sowohl die reinen Enantiomeren bzw. die einheitlichen Diastereomeren als auch die bei der Synthese üblicherweise anfallenden Mischungen wirksam.

Für die Herstellung von Ausgangsverbindungen der Formeln II und III können beispielsweise die folgenden Dibenzofuran-3-ol eingesetzt werden:

Dibenzofuran-3-ol, 7-Fluordibenzofuran-3-ol, 7-Chlordibenzofuran-3-ol, 2,4,7-Trichlordibenzofuran-3-ol, 5-Chlordibenzofuran-3-ol, 6-Chlordibenzofuran-3-ol, 8-Chlordibenzofuran-3-ol, 5,7-Dichlorbenzofuran-3-ol, 6,7-Dichlordibenzofuran-3-ol, 7,8-Dichlordibenzofuran-3-ol, 7-Bromdibenzofuran-3-ol, 7-Methyldibenzofuran-3-ol, 6,8-Dichlor-7-methyldibenzofuran-3-ol, 6-Trifluormethyldibenzofuran-3-ol, 7-Trifluormethyldibenzofuran-3-ol, 7-tert.-Butyldibenzofuran-3-ol, 7-Methoxydibenzofuran-3-ol, 7-Chlor-2-nitrodibenzofuran-3-ol und 7-Ethoxydibenzofuran-3-ol.

Die Beispiele 1 bis 52 erläutern die Herstellung der Verbindungen der Formel I.

*Beispiel 1:*

Zu einer Lösung von 262 g (1,2 Mol) 7-Chlordibenzofuran-3-ol in 600 ml trockenem Dimethylformamid wurden 83 g (0,6 Mol) Kaliumcarbonat zugegeben und anschliessend bei 100° C eine Lösung von 212 g (2,55 Mol) Ethylencarbonat in 300 ml Dimethylformamid in 90 Minuten zugetropft. Das Gemisch wurde 8 Stunden bei 120° C gerührt, bei 100° C abgesaugt und das Filtrat eingeengt. Der Rückstand wurde in 2,5 l Essigester gelöst, dreimal mit je 300 ml Wasser gewaschen, die organische Schicht getrocknet, mit 10 g Tierkohle entfärbt, auf 500 ml eingeengt und auf 10° C abgekühlt. Der Niederschlag wurde abgesaugt und nacheinander bei +5° C mit 60 ml Methanol, 50 ml Ether und 200 ml Petrolether gewaschen und getrocknet. Man erhielt 225 g 2-(7-Chlordibenzofuran-3-oxy)ethanol vom Schmp. 118 bis 120° C; Ausbeute 71,4% d. Th.

Zu einer Suspension von 26,2 g (0,1 Mol) 2-(7-Chlordibenzofurany-3-oxy)ethanol in 100 ml trockenem Toluol wurden 11,9 g (0,15 Mol) Pyridin zugesetzt und anschliessend bei 3 bis 15° C 17,9 g (0,15 Mol) Thionylchlorid zugetropft. Das Gemisch wurde 1 Tag bei 40° C und weitere 8 Stunden bei 70° C gerührt, abgekühlt und mit 150 ml Toluol und 200 ml Eiswasser versetzt. Die organische Schicht wurde nacheinander mit 100 ml Wasser, 50 ml Salzsäure, 50 ml 1n Natronlauge und schliesslich mit 100 ml Wasser gewaschen, getrocknet und eingeengt. Der feste Rückstand wurde mit 40 ml Petrolether bei +3° C (Eisbad) 30 Minuten verrührt, abgesaugt, mit wenig

kaltem Petrolether gewaschen und getrocknet. Man erhielt 22,8 g 1-Chlor-2-(7-chlordibenzofurany-3-oxy)ethan als farblose Kristalle vom Schmp. 105 bis 107° C; Ausbeute 81% d. Th.

19,6 g (0,06 Mol) 1-Chlor-2-(7-chlordibenzofuran-3-oxy)ethan und 16,4 g (0,24 Mol) Imidazol in 50 ml Dimethylformamid wurden mit 0,5 g Kaliumiodid versetzt und 16 Stunden bei 110° C gerührt, abgekühlt und anschliessend unter vermindertem Druck eingeengt. Der Rückstand wurde zwischen 100 ml Wasser und 1 l Methylenchlorid verteilt und unter Eiskühlung mit 20 ml 50%iger Natronlauge versetzt. Die organische Schicht wurde abgetrennt, dreimal mit je 50 ml Wasser gewaschen, getrocknet und im Vakuum eingeengt. Der feste, farblose Rückstand wurde mit 15 ml Ether bei +5° C 1 Stunde stehenlassen, abgesaugt und mit 20 ml kaltem n-Pentan gewaschen. Man erhielt 13,2 g (70,4% d. Th.) N-2-(7-Chlordibenzofuran-3-oxy)ethylimidazol vom Schmp. 150 bis 151° C.

Eine Lösung von 15,6 g (0,05 Mol) N-2-(7-Chlordibenzofuran-3-oxy)ethylimidazol und 17,1 g (0,1 Mol) Benzylbromid in 40 ml trockenem Dioxan und 30 ml trockenem Acetonitril wurde 2 Tage bei 70° C gerührt und auf 0° C abgekühlt. Der kristalline Niederschlag wurde abgesaugt, mit 50 ml trockenem Ether gewaschen und unter vermindertem Druck getrocknet. Man erhielt 19,7 g entsprechend einer Ausbeute von 81,5% d. Th., $N^1$-Benzyl-$N^3$-2-(7-chlordibenzofuran-3-oxy)ethylimidazoliumbromid als weisse Kristalle vom Schmp. 191 bis 193° C.

*Beispiel 2:*

Eine Mischung aus 98,4 g (0,45 Mol) 7-Chlordibenzofuran-3-ol, 200 ml Methylethylketon, 93 g (0,67 Mol) Kaliumcarbonat und 283 g (1,4 Mol) Dibrompropan wurden 24 Stunden unter Rückfluss gerührt. Nach Absaugen des anorganischen Niederschlages wurde das Filtrat unter vermindertem Druck eingeengt. Der Rückstand wurde in 500 ml Methylenchlorid aufgelöst, nacheinander mit 100 ml Wasser, 100 ml 2n Natronlauge und 100 ml Wasser gewaschen, über Natriumsulfat getrocknet und unter vermindertem Druck eingeengt. Der feste Rückstand wurde mit 30 ml Pentan bei 0° C 30 Minuten stehenlassen, abgesaugt und mit 30 ml kaltem n-Pentan gewaschen. Man erhielt 77 g reines 1-Brom-3-(7-chlordibenzofuran-3-oxy)propan vom Schmp. 83 bis 85° C; Ausbeute 50% d. Th.

Die Lösung von 14 g (0,041 Mol) 1-Brom-3-(7-chlordibenzofuran-3-oxy)propan und 7,9 g (0,05 Mol) N-Benzylimidazol in 25 ml trockenem Dioxan und 25 ml trockenem Acetonitril wurden 40 Stunden bei 70° C gerührt. Nach dem Abkühlen auf 5° C (Eisbad) wurde der gebildete, kristalline Niederschlag abgesaugt, mit 30 ml Ether und schliesslich mit 50 ml n-Pentan gewaschen und getrocknet. Man erhielt 15 g, entsprechend einer Ausbeute von 73,2% d. Th. $N^1$-Benzyl-$N^3$-3-(7-chlordibenzofuran-3-oxy)propylimidazoliumbromid vom Schmp. 162 bis 166° C.

Analog den Beispielen 1 und 2 lassen sich beispielsweise folgende Verbindungen der Formel I herstellen:

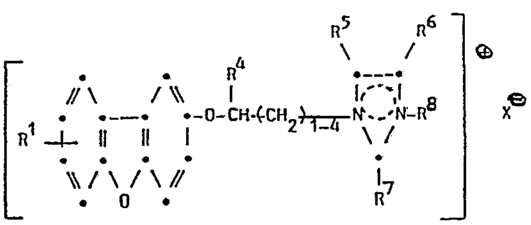

| Nr. | $R^1$ | $R^4$ | m | $R^5$ | $R^6$ | $R^7$ | $R^8$ | X | Fp. (° C) |
|---|---|---|---|---|---|---|---|---|---|
| 3 | H | H | 1 | H | H | H | $4-Cl-C_6H_4-CH_2-$ | Br | 177-179 |
| 4 | H | H | 1 | H | H | H | $CH_3-$ | $No_3$ | |
| 5 | 7-Cl | H | 1 | H | H | H | $1-C_{10}H_7-CH_2-$ | Cl | 225-227 |
| 6 | 7-Cl | H | 1 | H | H | H | $4-F-C_6H_4-CH_2-$ | Br | 218-220 |
| 7 | 7-Cl | H | 1 | H | H | $CH_3$ | $4-ClC_6H_4-CH_2-$ | Br | |
| 8 | 7-Cl | H | 1 | H | H | H | $4-ClC_6H_4-CH_2$ | Br | 232-234 |
| 9 | 7-Cl | H | 1 | H | H | H | $4-BrC_6H_4-CH_2-$ | Br | 225-227 |
| 10 | 7-Cl | H | 1 | H | H | H | $2,4-Cl_2C_6H_3-CH_2-$ | Cl | 212-215 |
| 11 | 7-Cl | H | 1 | H | H | H | $4-(CH_3)C_6H_4-CH_2-$ | Br | 210-212 |
| 12 | 7-Cl | H | 1 | H | H | H | $4-(tert.-C_4H^9)C_6H_4-CH_2-$ | Br | 180-182 |
| 13 | 7-Cl | H | 1 | H | H | H | $4-(CF_3)C_6H_4-CH_2-$ | Br | 224-226 |
| 14 | 7-Cl | H | 1 | H | H | H | $4-(NO_2)C_6H_4-CH_2-$ | Br | 243-245 |
| 15 | 7-Cl | H | 1 | H | H | H | $4-(CN)C_6H_4-CH_2-$ | Br | |
| 16 | 7-Cl | H | 1 | H | H | H | $CH_2=CH-CH_2-$ | Br | 145-147 |
| 17 | 7-Cl | H | 1 | H | H | H | $CH_3-CH=CH-CH_2-$ | Br | 156-159 |

| Nr. | $R^1$ | $R^4$ | m | $R^5$ | $R^6$ | $R^7$ | $R^8$ | X | Fp. (°C) |
|---|---|---|---|---|---|---|---|---|---|
| 18 | 7-Cl | H | 1 | H | H | H | $n-C_4H_9$ | Br | 155-158 |
| 19 | 7-Cl | H | 1 | H | H | H | $HC\equiv C-CH_2-$ | Br | |
| 20 | 7-Cl | $CH_3$ | 1 | H | H | H | $C_2H_5-$ | I | |
| 21 | 7-Cl | $CH_3$ | 1 | H | H | H | $C_6H_5-CH_2-$ | Br | 146-149 |
| 22 | 7-Cl | $CH_3$ | 1 | H | H | H | $4-(CH_3)C_6H_4-CH_2-$ | Br | 175-178 |
| 23 | 7-Cl | $CH_3$ | 1 | H | H | H | 4-(tert.-$C_4H_9$)$C_6H_4-CH_2-$ | Br | 195-197 |
| 24 | 7-Cl | $CH_3$ | 1 | H | H | H | $2,4-Cl_2C_6H_3-CH_2-$ | Cl | 179-181 |
| 25 | 7-Cl | $CH_3$ | 1 | H | H | H | $2,4-Cl_2C_6H_3-O-CH_2-CH_2-$ | Br | 145-146 |
| 26 | 7-Cl | $CH_3$ | 1 | H | H | H | $3-(NO_2)C_6H_4-O-CH_2-CH_2-$ | Br | Harz |
| 27 | 7-Cl | H | 1 | H | H | H | $C_6H_5-O-CH_2-CH_2-$ | Br | 169-171 |
| 28 | 7-Cl | H | 1 | H | H | H | $3-(CH_3)C_6H_4-O-CH_2-CH_2-$ | Br | 166-168 |
| 29 | 7-Cl | H | 1 | H | H | H | $3-(CF_3)C_6H_4-O-(CH_2)_4-$ | Br | 116-121 |
| 30 | 7-Cl | H | 1 | H | H | H | $4-(NO_2)C_6H_4-O-CH_2-CH_2-$ | Br | |
| 31 | 7-Cl | H | 2 | H | H | H | $4-ClC_6H_4-CH_2-$ | Br | 164-166 |
| 32 | 7-Cl | H | 2 | H | H | H | $4-BrC_6H_4-O-CH_2-CH_2-$ | Br | 127-131 |
| 33 | 7-Cl | H | 2 | H | H | H | $4-FC_6H_4-O-CH_2-CH_2-$ | Br | Harz |
| 34 | 7-Cl | H | 2 | H | H | H | $1-C_{10}H_7-O-CH_2-CH_2-$ | Br | Harz |
| 35 | 7-Cl | H | 2 | H | H | H | $2-C_{10}H_7-O-CH_2-CH_2-$ | Br | Harz |
| 36 | 7-Cl | H | 3 | H | H | H | $HC\equiv C-CH_2-$ | Br | 136-138 |
| 37 | 7-Cl | $CH_3$ | 3 | H | H | H | $C_6H_5-CH_2-$ | Br | Harz |
| 38 | 7-Cl | H | 3 | H | H | H | $C_6H_5-CH_2-$ | Br | 138-142 |
| 39 | 7-Cl | H | 3 | H | H | H | $4-ClC_6H_4-CH_2-CH_2-$ | Br | 196-199 |
| 40 | 7-Cl | H | 3 | H | H | H | $4-(CH_3)C_6H_4-CH_2-$ | Br | 168-172 |
| 41 | 7-Cl | H | 3 | H | H | H | 4-(tert.-$C_4H_9$)$C_6H_4-CH_2-$ | Br | Harz |
| 42 | 7-Cl | H | 1 | H | H | H | $C_6H_5-CH=CH-CH_2-$ | Br | 176-178 |
| 43 | 7-Cl | H | 1 | H | H | H | $CH_3$<br>\|<br>$C_6H_5-CH=C-CH_2-$ | Br | |
| 44 | 7-Cl | H | 1 | H | H | H | $CH_3$<br>\|<br>4-(tert.-$C_4H_9$)$C_6H_4-CH=C-CH_2-$ | Br | 168-170 |
| 45 | 7-Cl | H | 1 | H | H | H | $4-(CH_3O)C_6H_4-CH=CH-CH_2-$ | Br | |
| 46 | $7-CH_3$ | H | 1 | H | H | H | $C_6H_5-O-(CH_2)_4-$ | Br | |
| 47 | $7-CH_3$ | H | 1 | H | H | H | $4-ClC_6H_4-CH_2-$ | Br | |
| 48 | 7-F | H | 1 | H | H | $CH_3$ | $C_6H_5-CH_2-$ | Cl | |
| 49 | 7-F | H | 3 | H | H | H | $4-ClC_6H_4-CH_2-$ | Br | 158-160 |
| 50 | $6-CF_3$ | H | 1 | H | H | H | $4-ClC_6H_4-CH_2-$ | Br | 213-215 |
| 51 | $6,8-Cl_2$, $7-CH_3$ | H | 3 | H | H | H | $4-ClC_6H_4-CH_2-$ | Br | 210-212 |
| 52 | $6,7-Cl_2$ | H | 1 | H | H | H | $4-ClC_6H_4-CH=CH-CH_2-$ | Br | |

Die neuen Wirkstoffe zeigen eine starke fungitoxische Wirksamkeit gegen phytopathogene Pilze. Sie dienen insbesondere zur Verhütung und Heilung von Pflanzenkrankheiten, die durch Mikroorganismen verursacht werden, wie z. B. *Botrytis begoniae, Botrytis cinerea, Plasmopara viticola, Monila fructigena, Alternaria solani, Solerotinia solerotiorum, Piricularia oryzae, Pellicularia filamentosa, Erysiphe graminis, Erysiphe cichoriacearum, Chaetomium globosum, Sclerotinia cinerea, Aspergillus niger, Xanthomonas oryzae, Xanthomonas citri, Phytophthora infestans* (an Kartoffeln und Tomaten).

Die erfindungsgemässen Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 bis 5 kg Wirkstoff je Hektar.

Die erfindungsgemässen Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrösserung des fungiziden Wirkungsspektrums; bei einer Anzahl dieser Fungizidmischungen treten auch synergistische Effekte auf, d. h. die fungizide Wirksamkeit des Kombinationsproduktes ist grösser als die der addierten Wirksamkeiten der Einzelkomponenten. Eine besonders günstige Vergrösserung des Wirkungsspektrums wird mit folgenden Fungiziden erzielt:

Manganethylenbisdithiocarbamat
Manganzinkethylenbisdithiocarbamat
Ammoniakkomplex von Zink-(N,N-ethylenbisdithiocarbamat)
N-Trichlormethylthiotetrahydrophthalimid
N-Trichlormethylthiophthalimid
5-Ethoxy-3-trichlormethyl-1,2,3-thiadiazol
2-Methoxycarbonylaminobenzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäureanilid
2,4,5-Trimethylfuran-3-carbonsäureanilid
2-Methylfuran-3-carbonsäureanilid
2,5-Dimethylfuran-3-carbonsäurecyclohexylamid
N-Cyclohexyl-N-methoxy-2,5-dimethylfuran-3-carbonsäureamid
5-Methyl-5-vinyl-3-(3,5-dichlorphenyl)-2,4-dioxo)-1,3-oxazolidin
3-(3,5-Dichlorphenyl)-5-methyl-5-methoxymethyl-1,3-oxazolidin-2,4-dion

Die erfindungsgemässen Verbindungen können aber auch mit folgenden Fungiziden kombiniert werden:

Dithiocarbamate und deren Derivate, wie
Ferridimethylthiocarbamat
Zinkdimethyldithiocarbamat
Zinkethylenbisdithiocarbamat
Tetramethylthiuramdisulfide
Zink-(N,N-propylenbisdithiocarbamat)
Ammoniakkomplex von Zink-(N,N'-propylenbisdithiocarbamat), und
N,N'-Polypropylenbis(thiocarbamoyl)disulfid
Nitroderivate, wie
Dinitro-(1-methylheptyl)phenylcrotonat
2.sek.-Butyl-4,6-dinitrophenyl-3,3-dimethylacrylat
2-sek.-Butyl-4,6-dinitrophenylisopropylcarbonat
Heterocyclische Strukturen, wie
2-Heptadecyl-2-imidazolinacetat
2,4-Dichlor-6-(o-chloranilino)-s-triazin
O,O-Diethylphthalimidophosphonothioat
5-Amino-1-(bis(dimethylamino)phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazolcarbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathin
2-(Furyl-(2))-benzimidazol
Piperazin-1,4-diylbis-(1-(2,2,2-trichlorethyl))-formamid
2-(Thiazolyl-(4))-benzimidazol
5-Butyl-2-dimethylamino-4-hydroxy-6-methylpyrimidin
Bis-(p-chlorphenyl)-3-pyridinmethanol
1,2-Bis-(3-ethoxycarbonyl-2-thioureido)benzol
1,2-Bis-(3-methoxycarbonyl-2-thioureido)benzol sowie
verschiedene Fungizide, wie
Dodecylguanidinacetat
3-(3-(3,5-Dimethyl-2-oxycyclohexyl)-2-hydroxyethyl)glutarimid
Hexachlorbenzol
N-Dichlorfluormethylthio-N',N'-dimethyl-N-phenylschwefelsäurediamid
2,5-Dimethylfuran-3-carbonsäureanilid
2-Methylbenzoesäureanilid
2-Jodbenzoesäureanilid
1-(3,4-Dichloranilino)-1-formylamino-2,2,2-trichlorethan
2,6-Dimethyl-N-tridecylmorpholin bzw. dessen Salze
2,6-Dimethyl-N-cyclododecylmorpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidinmethanol
DL-Methyl-N-(2,6-dimethylphenyl)-N-furoyl-(2)-alaninat
DL-N-(2,6-Dimethylphenyl)-N-(2'-methoxyacetyl)alaninmethylester
5-Nitroisophthalsäurediisopropylester
N-(2,6-Dimethylphenyl)-N-chloracetyl-D,L-2-aminobutyrolacton
N-(n-Propyl)-N-(2,4,6-trichlorphenoxyethyl)-N'-imidazolylharnstoff
N-Cyclohexyl-N-methoxy-2,5-dimethylfuran-3-carbonsäureamid
2,4,5-Trimethylfuran-3-carbonsäureanilid
5-Methyl-5-vinyl-3-(3,5-dichlorphenyl)-2,4-dioxo-1,3-oxazolidin
5-Methoxymethyl-5-methyl-3-(3,5-dichlorphenyl)-2,4-dioxo-1,3-oxazolidin
N-(3-(p-tert.-Butylphenyl)-2-methylpropyl)-cis-2,6-dimethylmorpholin
N-Formyl-N-morpholin-2,2,2-trichlorethylacetal

1-(2-(2,4-Dichlorphenyl)-4-ethyl-1,3-dioxolan-2-yl-methyl)-1H-1,2,4-triazol
1-(2-(2,4-Dichlorphenyl)-4-n-propyl-1,3-dioxolan-2-yl-methyl)-1H-1,2,4-triazol
2-(Thiocyanomethylthio)benzthiazol
1-(2-(2,4-Dichlorphenyl)-2-(2-propenyloxy)-ethyl)-1H-imidazol
2,4,5,6-Tetrachlorisophthalodinitril
Methylenbisthiocyanat
Tributylzinnoxid
Mercaptobenzthiazol
Benzisothiazolon und seine Alkalisalze
Alkaliverbindungen des N'-Hydroxy-N-cyclohexyldiazeniumoxids
2-(Methoxycarbonylamino)benzimidazol
2-Methyl-3-oxo-5-chlorthiazolin-3-on
Trihydroxymethylnitromethan
Glutardialdehyd
Chloracetamid

Die neuen Wirkstoffe werden beispielsweise in Form von direkt versprühbaren Lösungen, Suspensionen, Emulsionen, auch in Form von hochprozentigen wässerigen, öligen oder sonstigen Dispersionen, Pasten, Stäubemitteln, Streumitteln, Granulaten durch Versprühen, Verstäuben, Verstreuen, Verstreichen oder Giessen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitteln in Wasser homogenisiert werden. Es können aber auch aus wirksamer Substanz, Netz-, Haft-, Dispergier- oder Emulgiermittel und eventuell Lösungsmittel oder aus Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxidkondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löss, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

Beispiele für solche Pflanzenschutzmittelzubereitungen sind:

I. Man vermischt 90 Gewichtsteile der Verbindung des Beispiels 1 mit 100 Gewichtsteilen N-Methylpyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässerige Dispersion, die 0,02 Gew.-% des Wirkstoffs enthält.

III. 20 Gewichtsteile der nach Beispiel 13 erhältlichen Verbindung werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und Verrühren der Lösung in 100 000 Gewichtsteilen Wasser erhält man eine wässerige Dispersion, die 0,02 Gew.-% des Wirkstoffs enthält.

IV. 20 Gewichtsteile der nach Beispiel 14 erhältlichen Verbindung werden in einer Mischung gelöst, die aus 25 Gewichtsteilen Cyclohexanol,

65 Gewichtsteilen einer Mineralölfraktion vom Siedepunkt 210 bis 280° C und 10 Gewichtsteilen des Anlagerungsproduktes von 40 Mol Ethylenoxid an 1 Mol Ricinusöl besteht. Durch Eingiessen und Verrühren der Lösung in 100 000 Gewichtsteilen Wasser erhält man eine wässerige Dispersion, die 0,02 Gew.-% des Wirkstoffs enthält.

V. 20 Gewichtsteile der nach Beispiel 21 erhältlichen Verbindung werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 5 Gewichtsteile der nach Beispiel 25 erhältlichen Verbindung werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der nach Beispiel 37 erhältlichen Verbindung werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der nach Beispiel 42 erhältlichen Verbindung werden mit 30 Teilen Natriumsalz eines Phenolsulfonsäure/Harnstoff/Formaldehyd-Kondensates, 2 Teilen Kieselgel und 48 Teilen Wasser innig vermischt. Man erhält eine stabile wässerige Dispersion.

IX. 20 Teile der nach Beispiel 51 erhältlichen Verbindung werden mit 2 Teilen Calciumsalz der Dodecylbenzolsulfonsäure, 8 Teilen Fettalkoholpolyglykolether, 2 Teilen Natriumsalz eines Phenolsulfonsäure/Harnstoff/Formaldehyd-Kondensates und 68 Teilen eines paraffinischen Mineralöls innig vermischt. Man erhält eine stabile ölige Dispersion.

Auch für technische Zwecke, z. B. als Holzschutzmittel, sind die erfindungsgemässen Imidazoliumsalze geeignet. Über die fungizide Wirkung hinaus wurde auch eine bakterizide Wirksamkeit der Verbindungen festgestellt, so dass sie auch als solche im Pflanzenschutz und als technische Mikrozide in Betracht kommen. Beispielsweise können folgende Mikroorganismen damit bekämpft werden.

*Staphylococcus aureus, Escherichia coli, Klebsiella pneumoniae, Citrobacter freundii, Proteus vulgaris, Pseudomonas aeruginosa, Pseudomonas fluorescens, Xanthomonas vesicatoria, Xanthomonas malvaccarum, Erwinia carotovora, Erwinia amylovora, Desulfovibrio desulfuricans, Streptoverticillium rubrireticuli, Aspergillus niger, Aspergillus versicolor, Penicillium funiculosum, Paecilomyces variotii, Trichoderma viride, Chaetomium globosum, Candida albicans, Geotrichum candidans, Monilia sitophila, Scenedesmus quadricauda, Chlorella vulgaris, Nostoc muscorum.*

Die üblichen Anwendungskonzentrationen betragen 0,01 bis 1% Wirkstoff, bezogen auf das Gewicht des zu schützenden Materials; beim Einsatz zur Wasserbehandlung bei der Erdölförderung, in Schwimmbädern, Rückkühlwerken, Luftbefeuchtungsanlagen, Blumenfrischhaltemitteln oder in der Papierindustrie sind Wirkstoffmengen von 5 bis 100 ppm ausreichend, um eine Mikroorganismenentwicklung zu unterdrücken. Gebrauchsfertige Desinfektionsmittellösungen enthalten 0,2 bis 5% Wirkstoff.

Die Beispiele 53 bis 56 belegen die biologische Wirkung der neuen Verbindungen. Vergleichsmittel (Vglm.) ist stets der bekannte, insbesondere zur Bekämpfung von *Botrytis* geeignete Wirkstoff N-Trichlormethylthiotetrahydrophthalimid („Chem. Week.", 21. Juni 1972, S. 46).

*Beispiel 53:*

*Wirksamkeit gegen* Botrytis cinerea *an Paprika*

Paprikasämlinge der Sorte Neusiedler Ideal Elite werden, nachdem sich 4 bis 5 Blätter gut entwickelt haben, mit 0,05%igen wässerigen Suspensionen, die 80% Wirkstoff und 20% Emulgiermittel in der Trockensubstanz enthalten, tropfnass gespritzt. Nach dem Antrocknen des Spritzbelages werden die Pflanzen mit einer Konidienaufschwemmung des Pilzes *Botrytis cinerea* besprüht und bei 22 bis 24° C in eine Kammer mit hoher Luftfeuchtigkeit gestellt. Nach 5 Tagen hat sich die Krankheit auf den unbehandelten Kontrollpflanzen so stark entwicklet, dass die entstandenen Blattnekrosen den überwiegenden Teil der Blätter bedecken.

Bewertung:
0 = kein Pilzbefall, abgestuft bis 5 = Totalbefall.

| Wirkstoff Nr. | Befall |
|---|---|
| 5 | 0 |
| 6 | 1 |
| 8 | 1 |
| 9 | 0 |
| 10 | 0 |
| 11 | 0 |
| 12 | 0 |
| 13 | 1 |
| 14 | 0 |
| 22 | 2 |
| 23 | 0 |
| 24 | 1 |
| 25 | 1 |
| 26 | 0 |
| 32 | 1 |
| 33 | 1 |
| 35 | 0 |

| Wirkstoff Nr. | Befall |
|---|---|
| 41 | 1 |
| 44 | 1 |
| Vglm. | 3 |
| Unbehandelt | 5 |

*Beispiel 54:*

*Wirksamkeit gegen* Phytophthora infestans *an Tomaten*

Blätter von Topfpflanzen der Sorte Grosse Fleischtomate werden mit 0,025%iger wässeriger Spritzbrühe, die 80% Wirksstoff und 20% Emulgiermittel in der Trockensubstanz enthält, besprüht. Nach dem Antrocknen des Spritzbelages werden die Blätter mit einer Zoosporenaufschwemmung des Pilzes *Phytophthora infestans* infiziert. Die Pflanzen werden dann in einer wasserdampfgesättigten Kammer bei Temperaturen zwischen 16 und 18° C aufgestellt. Nach 5 Tagen hat sich die Krankheit auf den unbehandelten, jedoch infizierten Kontrollpflanzen so stark entwikkelt, dass die fungizide Wirksamkeit der Substanzen beurteilt werden kann.

Bewertung:

0 = kein Pilzbefall, abgestuft bis 5 = Totalbefall.

| Wirkstoff Nr. | Befall |
|---|---|
| 5 | 2 |
| 6 | 1 |
| 9 | 0 |
| 10 | 0 |
| 14 | 1 |
| 21 | 0 |
| 22 | 1 |
| 24 | 2 |
| 25 | 1 |
| 26 | 2 |
| 32 | 1 |
| 36 | 2 |
| 38 | 2 |
| 39 | 1 |
| 40 | 1 |
| 41 | 1 |
| 44 | 1 |
| Vglm. | 3 bis 4 |
| Unbehandelt | 5 |

*Beispiel 55:*

Zur Prüfung der Wirksamkeit gegenüber Pilzen werden die Wirkstoffe einer Nährlösung für das Wachstum der Pilze *Aspergillus niger, Oidium lactis* bzw. *Candida albicans* in Mengen von 200, 100, 50, 25, 12, 6 und 3 Gewichtsteilen pro Million Teile Nährlösung zugesetzt. Jeweils 10 ml Nährlösungs/Wirkstoff-Gemisch werden in sterile Reagenzgläser gegeben und mit einem Tropfen einer Sporensuspension beimpft, die $10^6$ Konidien bzw. Zellen enthält. Nach 120stündiger Bebrütung werden aus denjenigen Röhrchen, die kein sichtbares Pilzwachstum zeigen, Proben entnommen und auf Pilznährböden übertragen. In der Tabelle wird die höchste Verdünnunggstufe, bei welcher kein Wachstum der Pilze mehr erfolgt, also die minimale Hemmstoffkonzentration, angegeben.

*(Tabelle am Ende des Patents)*

*Beispiel 56:*

Zur Ermittlung der Wirksamkeit der neuen Verbindungen gegenüber Bakterien werden zu je 5 ml zunehmend verdünnter Lösungen der Wirkstoffe 5 ml Nährbouillon in sterile Reagenzgläser gegeben und vermischt. Durch Zugabe von einem Tropfen einer 1:10 verdünnten 16 Stunden alten Bouillonkultur der Bakterienarten *Staphylococcus aureus* bzw. *Escherichia coli* oder *Proteus vulgaris* werden dann die Röhrchen beimpft und 24 Stunden bei 37° C bebrütet. Nach dieser Zeit werden Proben aus den Röhrchen auf Bakteriennährböden übertragen und diese ebenfalls 24 Stunden lang bei 37° C bebrütet. Die höchste Verdünnungsstufe, bei welcher noch keine Bakterienentwicklung erfolgt, wird als minimale Hemmstoffkonzentration angegeben.

*(Tabelle am Ende des Patents)*

**Patentansprüche**

1. Dibenzofuranoxyalkylimidazoliumsalze der Formel (I)

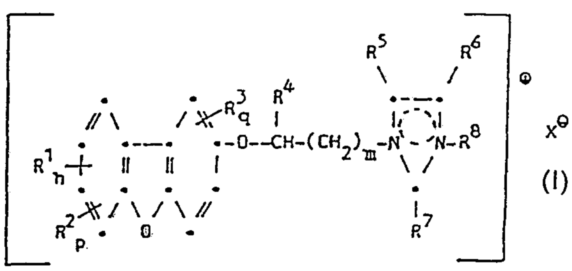

(I)

in der

$R^1$, $R^2$ und $R^3$ gleich oder verschieden sind und Wasserstoff, Halogen, eine ggf. halogensubstituierte Alkyl- oder Alkoxygruppe mit 1 bis 4 Kohlenstoffatomen, höchstens einer der drei Reste eine Cyan- oder Nitrogruppe,

$R^4$, $R^5$, $R^6$ und $R^7$ unabhängig voneinander Wasserstoff oder Alkyl mit 1 bis 4 Kohlenstoffatomen bedeuten, und

$R^8$ einen Alkyl-, Alkenyl- oder Alkinylrest mit 1 bis 6 Kohlenstoffatomen oder eine der Gruppen

$$-CH_2-Ar, \; -CH_2-\underset{\displaystyle R^4}{\underset{|}{C}}=CH-Ar$$

oder $-(CH_2)_{2\text{-}5}-O-Ar$
bedeutet, wobei Ar für 1- und 2-Naphthyl, Biphenylyl oder Phenyl steht und der Phenylrest durch Fluor, Chlor, Brom, Nitro, Trifluormethyl, Cyan oder Alkyl, Alkenyl und Alkoxy mit bis zu 5 Kohlenstoffatomen substituiert sein kann,

m die Zahlen 1, 2, 3 und 4,

n, p und q die Zahlen 0, 1, 2 oder 3, und

X das Anion einer einbasigen, nichtphytotoxischen Säure oder ein Äquivalent einer mehrbasigen, nichtphytotoxischen Säure bedeuten.

2. Dibenzofuranoxyalkylimidazoliumsalze gemäss Anspruch 1, dadurch gekennzeichnet, dass

$R^1$ Halogen, eine Alkyl- oder Alkoxygruppe mit 1 bis 4 Kohlenstoffatomen, Trifluormethyl oder Nitro,

$R^2$, $R^3$ Wasserstoff,

m die Zahlen 1, 2, 3 und 4,

n die Zahlen 0, 1, 2 oder 3,

p und q 0,

$R^4$, $R^5$, $R^6$ und $R^7$ unabhängig voneinander Wasserstoff, Methyl oder Ethyl,

$R^8$ Methyl, Ethyl, n-Propyl, n-Butyl, Isobutyl, n-Pentyl, Isopentyl, n-Hexyl, Vinyl, Allyl, 2-Buten-1-yl, 3-Methyl-2-buten-1-yl, Propargyl, oder die Reste

$$-CH_2-Ar,\ -CH_2-\underset{\displaystyle R^4}{\underset{|}{C}}=CH-Ar$$

oder $-(CH_2)_{2\text{-}5}-O-Ar$
wobei Ar für 1- und 2-Naphtyl, Biphenylyl, Phenyl, 4-Fluorphenyl, 3- und 4-Chlorphenyl, 4-Bromphenyl, 3,4-Dichlorphenyl, 2,4-Dichlorphenyl, 2,6-Dichlorphenyl, 2-Methyl-4-chlorphenyl, 4-Methoxyphenyl, 4-Ethoxyphenyl, 4-Methylphenyl, 4-Isopropylphenyl, 4-tert.-Butylphenyl, 4-Nitrophenyl, 4-Trifluormethylphenyl, 2,4-Dimethylphenyl und 4-Cyanphenyl steht, und

X das Anion bzw. ein Äquivalent eines Anions einer nichtphytotoxischen Säure bedeuten.

3. Verfahren zur Herstellung von Dibenzofuranderivaten gemäss Anspruch 1, dadurch gekennzeichnet, dass man

a) Verbindungen der Formel (II)

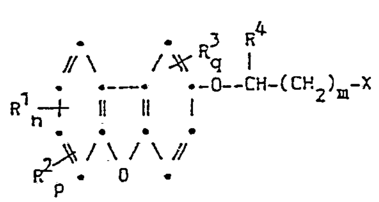

(II)

in der $R^1$, $R^2$, $R^3$, $R^4$, X, m, n, p und q die im Anspruch 1 angegebenen Bedeutungen haben, mit einem Imidazolderivat der Formel (III)

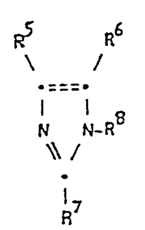

(III)

in der $R^5$, $R^6$, $R^7$ und $R^8$ die im Anspruch 1 angegebenen Bedeutungen haben, oder

b) Verbindungen der Formel (IV)

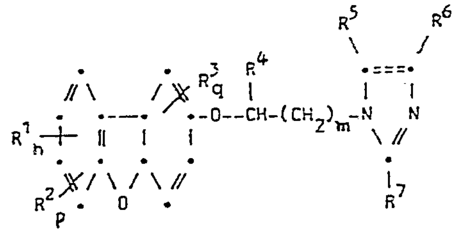

(IV)

in der $R^1$, $R^2$, $R^3$, $R^4$, $R^5$, $R^6$, $R^7$, m, n, p und q die im Anspruch 1 angegebenen Bedeutungen haben, mit einer Verbindung der Formel $R^8-X$, in der $R^8$ und X die im Anspruch 1 angegebenen Bedeutungen haben, umsetzt.

4. Pflanzenschutzmittel, enthaltend inerte Zusatzstoffe und als Wasserstoffe ein Dibenzofuranoxyalkylimidazoliumsalz gemäss Anspruch 1.

5. Verfahren zur Bekämpfung von Pilzen und Bakterien, dadurch gekennzeichnet, dass man ein Dibenzofuranoxyalkylimidazoliumsalz gemäss Anspruch 1 auf diese bzw. auf durch Bakterien- oder Pilzbefall bedrohte Flächen, Pflanzen, Saatgüter oder Holz einwirken lässt.

6. Verfahren zur Herstellung eines fungiziden oder bakteriziden Mittels gemäss Anspruch 4, dadurch gekennzeichnet, dass man ein Dibenzofuranderivat gemäss Anspruch 1 mit festen oder flüssigen Trägerstoffen sowie ggf. mit einem oder mehreren oberflächenaktiven Mitteln mischt.

## Claims

1. A dibenzofuranyloxyalkylimidazolium salt of the Formula (I):

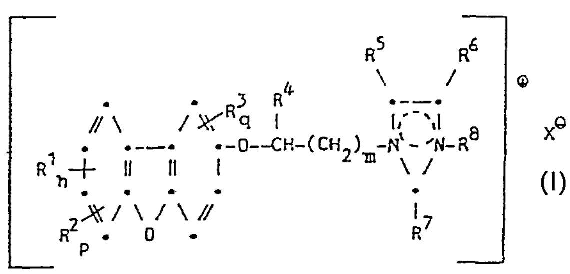

(I)

where

$R^1$, $R^2$ and $R^3$ are identical or different and are each hydrogen, halogen, unsubstituted or halogen-substituted alkyl or alkoxy, each of 1 to 4 carbon atoms, not more than one of the three radicals being cyano or nitro,

$R^4$, $R^5$, $R^6$ and $R^7$ independently of one another are hydrogen or alkyl of 1 to 4 carbon atoms,

$R^8$ is alkyl, alkenyl or alkynyl, each of 1 to 6 carbon atoms,

$$-CH_2-Ar,\ -CH_2-\overset{\displaystyle R^4}{\overset{|}{C}}=CH-Ar$$

or $-(CH_2)_{2\text{-}5}-O-Ar$,

where Ar is naphth-1-yl, naphth-2-yl, biphenylyl or phenyl, and the phenyl radical can be substituted by fluorine, chlorine, bromine, nitro, trifluoromethyl, cyano or alkyl, alkenyl or alkoxy of not more than 5 carbon atoms,

m is 1, 2, 3 or 4

n, p and q are each 0, 1, 2 or 3, and

X is an anion of a monobasic, non-phytotoxic acid or one equivalent of an anion of a polybasic, non-phytotoxic acid.

2. A dibenzofuranyloxyalkylimidazolium salt as claimed in Claim 1, where

$R^1$ is halogen, alkyl or alkoxy of 1 to 4 carbon atoms, trifluoromethyl or nitro,

$R^2$ and $R^3$ are each hydrogen,

m is 1, 2, 3 or 4,

n is 0, 1, 2 or 3,

p and q are each 0,

$R^4$, $R^5$, $R^6$ and $R^7$ independently of one another are hydrogen, methyl or ethyl,

$R^8$ is methyl, ethyl, n-propyl, n-butyl, isobutyl, n-pentyl, isopentyl, n-hexyl, vinyl, allyl, but-2-en-1-yl, 3-methyl-but-2-en-1-yl or propargyl, or is

$$-CH_2-Ar,\ -CH_2-\underset{}{\overset{R^4}{\overset{|}{C}}}=CH-Ar$$

or $-(CH_2)_{2\text{-}5}-O-Ar$,

Ar being naphth-1-yl, naphth-2-yl, biphenylyl, phenyl, 4-fluorophenyl, 3-chlorophenyl, 4-chlorophenyl, 4-bromophenyl, 3,4-dichlorophenyl, 2,4-dichlorophenyl, 2,6-dichlorophenyl, 2-methyl-4-chlorophenyl, 4-methoxyphenyl, 4-ethoxyphenyl, 4-methylphenyl, 4-isopropylphenyl, 4-tert.-butylphenyl, 4-nitrophenyl, 4-trifluoromethylphenyl, 2,4-dimethylphenyl or 4-cyanophenyl, and

X is an anion, or one equivalent of an anion, of a non-phytotoxic acid.

3. A process for the manufacture of dibenzofuran derivatives as claimed in Claim 1, wherein

(*a*) a compound of the Formula (II):

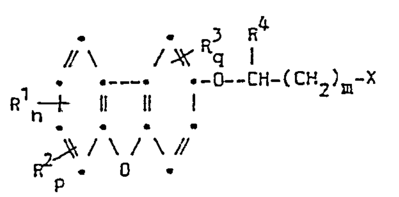

(II)

where

$R^1$, $R^2$, $R^3$, $R^4$, x, m, n, p and q have the meanings given in Claim 1, is reacted with an imidazole derivative of the Formula (III):

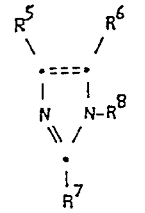

(III)

where

$R^5$, $R^6$, $R^7$ and $R^8$ have the meanings given in Claim 1, or

(*b*) a compound of the Formula (IV):

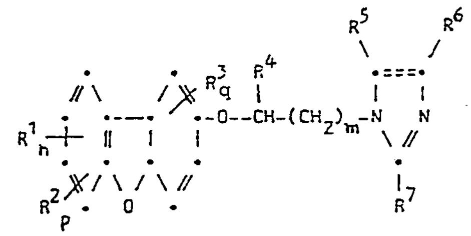

(IV)

where

$R^1$, $R^2$, $R^3$, $R^4$, $R^5$, $R^6$, $R^7$, m, n, p, and q have the meanings given in Claim 1, is reacted with a compound of the Formula $R^8-X$, where $R^8$ and X have the meanings given in Claim 1.

4. A crop protection agent containing inert additives and, as active ingredient, a dibenzofuranyloxyalkylimidazolium salt as claimed in Claim 1.

5. A process for combating fungi and bacteria, wherein a dibenzofuranyloxyalkylimidazolium salt as claimed in Claim 1 is allowed to act thereon, or on areas, plants, seed or wood threatened by bacterial of fungal attack.

6. A process for manufacturing a fungicidal or bactericidal agent as claimed in Claim 4, wherein a dibenzofuran derivative as claimed in Claim 1 is mixed with a solid or liquid carrier and, if desired, with one or more surfactants.

## Revendications

1. Sels de dibenzofurannoxyalkylimidazolium de la formule (I):

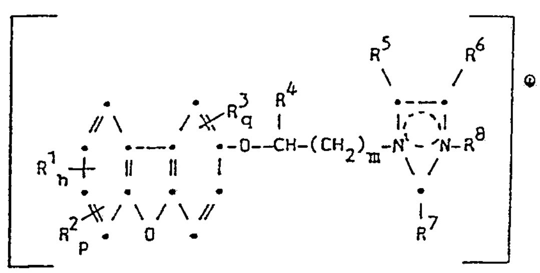

$X^{\ominus}$

(I)

dans laquelle:

$R^1$, $R^2$ et $R^3$, identiques ou différents, désignent chacun un atome d'halogène ou un radical alkyle ou alcoxy en $C_1$ à $C_4$ éventuellement halosubstitué, un seul de ces restes pouvant en outre désigner un groupe cyano ou nitro,

$R^4$, $R^5$, $R^6$ et $R^7$ désignent chacun, indépendamment l'un des autres, un atome d'hydrogène ou un radical alkyle en $C_1$ à $C_4$,

$R^8$ représente un radical alkyle, alcényle ou alcynyle en $C_1$ à $C_6$ ou l'un des groupes:

$$-CH_2-Ar,\ -CH_2-\underset{R^4}{\underset{|}{C}}=CH-Ar$$

et $-(CH_2)_{2\text{-}5}-O-Ar$,

où Ar désigne un groupe 1-naphtyle, 2-naphtyle, biphénylyle ou phényle, le groupe phényle pouvant être substitué par du fluor, du chlore ou du

brome ou des groupes nitro, trifluorométhyle ou cyano ou des radicaux alkyle, alcényle ou alcoxy avec jusqu'à cinq atomes de carbone,

m est un nombre valant 1, 2, 3 ou 4,

n, p et q sont des nombres valant 0, 1, 2 ou 3, et

X représente l'anion d'un acide monovalent non phytotoxique ou un équivalent d'un acide polyvalent non phytotoxique.

2. Sels de dibenzofurannoxyalkylimidazolium suivant la revendication 1, caractérisés en ce que:

$R^1$ représente un atome d'halogène, un radical alkyle ou alcoxy en $C_1$ à $C_4$ ou un groupe trifluorométhyle ou nitro,

m vaut 1, 2, 3 ou 4,

n vaut 0, 1, 2 ou 3,

p = q = 0,

$R^4$, $R^5$, $R^6$ et $R^7$ désignent chacun, indépendamment l'un des autres, un atome d'hydrogène ou un radical méthyle ou éthyle,

$R^8$ représente un groupe méthyle, éthyle, n-propyle, n-butyle, isobutyle, n-pentyle, isopentyle, n-hexyle, vinyle, allyle, butène-2 yle-1, méthyl-3 butène-2 yle-1 ou propargyle ou un groupe

$$-CH_2-Ar, \; -CH_2-\underset{\displaystyle R^4}{\underset{|}{C}}=CH-Ar$$

ou $-(CH_2)_{2\text{-}5}-O-Ar$,

où Ar représente un groupe naphtyle-1, naphtyle-2, biphénylyle ou phényle ou encore un groupe fluoro-4 phényle, chloro-3 ou chloro-4 phényle, bromo-4 phényle, dichloro-3,4 ou dichloro-2,4 ou dichloro-2,6 phényle, méthyl-2 chloro-4 phényle, méthoxy-4 phényle, éthoxy-4 phényle, méthyl-4 phényle, isopropyl-4 phényle, tert.-butyl-4 phényle, nitro-4 phényle, trifluorométhyl-4 phényle, diméthyl-2,4 phényle ou cyano-4 phényle, et

X est l'anion ou l'équivalent d'un anion d'un acide non phytotoxique.

3. Procédé de préparation de dérivés du dibenzofuranne suivant la revendication 1, caractérisé en ce que l'on fait réagir:

a) des composés de formule (II):

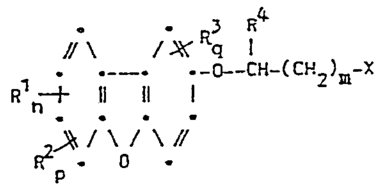

(II)

dans laquelle $R^1$, $R^2$, $R^3$, $R^4$, X, m, n, p et q possèdent les significations définies dans la revendication 1, avec un dérivé d'imidazole de la formule (III):

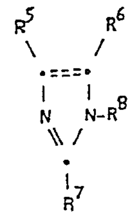

(III)

dans laquelle $R^5$, $R^6$, $R^7$ et $R^8$ possèdent les significations définies dans la revendication 1, ou

b) des composés de la formule (IV):

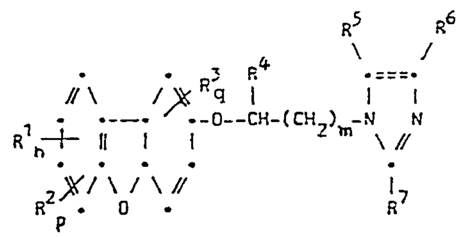

(IV)

dans laquelle $R^1$, $R^2$, $R^3$, $R^4$, $R^5$, $R^6$, $R^7$, m, n, p et q possèdent les significations définies dans la revendication 1, avec un composé de la formule $R^8-X$, dans laquelle $R^8$ et X possèdent les significations définies dans la revendication 1.

4. Composition phytosanitaire, contenant un sel de dibenzofurannoxyalkylimidazolium suivant la revendication 1 comme principe actif dans des additifs inertes.

5. Procédé de lutte contre des champignons et bactéries, caractérisé en ce que l'on laisse agir un sel de dibenzofurannoxyalkylimidazolium suivant la revendication 1 sur ceux-ci ou sur les surfaces, plantes, semences ou le bois risquant d'être attaqués par des bactéries ou champignons.

6. Procédé de préparation d'une composition à action fongicide ou bactéricide suivant la revendication 4, caractérisé en ce que l'on mélange un dérivé du dibenzofuranne suivant la revendication 1 avec des supports solides ou des véhicules liquides, auxquels on incorpore, le cas échéant, un ou plusieurs agents tensio-actifs.

| Wirkstoff | Minimale Hemmstoffkonzentration (Teile Wirkstoff pro Million Teile Nährlösung) | | |
|---|---|---|---|
| | *Aspergillus niger* | *Oidium lactis* | *Candida albicans* |
| 6 | 50 | 6 | 12 |
| 10 | 200 | 6 | 6 |
| 18 | 200 | 12 | 25 |
| 25 | 100 | 12 | 12 |
| 26 | 25 | 6 | 6 |
| 32 | 50 | 6 | 6 |
| 39 | 50 | 6 | 6 |

| Wirkstoff | Minimale Hemmstoffkonzentration (Teile Wirkstoff pro Million Teile Nährbouillon) | | |
|---|---|---|---|
| | *Staphylococcus aureus* | *Escherichia coli* | *Proteus vulgaris* |
| 6 | 6 | 6 | 12 |
| 10 | 6 | 6 | 6 |
| 18 | 6 | 50 | 25 |
| 25 | 6 | 12 | 12 |
| 26 | 6 | 25 | 25 |
| 32 | 6 | 12 | 12 |
| 39 | 12 | 12 | 12 |